# EUROPÄISCHE PATENTSCHRIFT

(11) **EP 2 391 414 B1**
(45) Veröffentlichungstag und Bekanntmachung des Hinweises auf die Patenterteilung: **10.04.2019**
(21) Anmeldenummer: 10701387.2
(22) Anmeldetag: 29.01.2010
(51) Int. Cl.: A61M 13/00, H05K 13/02

(54) **INSUFFLATOR**
INSUFFLATOR
INSUFFLATEUR

(30) Priorität: 29.01.2009 DE 102009007393
(43) Veröffentlichungstag der Anmeldung: 07.12.2011
(73) Patentinhaber: MGB Endoskopische Geräte GmbH Berlin, 12489 Berlin (DE); Universität Rostock, 18051 Rostock (DE)
(72) Erfinder: PAGEL, Lienhard, 18311 Klockenhagen (DE); GAßMANN, Stefan, 18059 Rostock (DE)
(74) Vertreter: ETL Wablat & Kollegen Patent- und Rechtsanwaltsgesellschaft mbH
(86) Internationale Anmeldenummer: PCT/EP2010/051095
(87) Internationale Veröffentlichungsnummer: WO 2010/086412

(56) Entgegenhaltungen:
- EP-A1- 1 596 455
- WO-A1-94/01154
- WO-A1-2007/114687
- DE-A1- 19 739 722
- US-A- 5 423 741
- US-B1- 6 299 592

## Beschreibung

Die Erfindung betrifft einen Insufflator.

Insufflatoren werden in der Laparoskopie (der endoskopischen Abdominalchirurgie) eingesetzt und dienen dazu, einem Operateur durch ein Endoskop eine freie Sicht auf ein Operationsfeld im Abdomen zu verschaffen, indem in das Abdomen Kohlendioxid (CO₂) eingeleitet wird. Hierzu wird über eine sog. Verres-Kanüle oder einen Trocar CO₂-Gas mit einem maximalen Druck von 30 mm_{Hg} in das Abdomen insuffliert. Durch den steigenden Innendruck wird die Bauchdecke (Perituneum) angehoben und im Abdomen entsteht der gewünschte Hohlraum, der die endoskopische Betrachtung des Operationsfeldes erlaubt. Derartige CO₂-Insufflatoren sind grundsätzlich bekannt.

Aus dem Stand der Technik beim Bau von Insufflatoren sind zwei grundlegende Prinzipien bekannt.

Gemäß dem ersten Prinzip sind die pneumatischen Bauteile am Gehäuse des Gerätes fest verschraubt. Die Verbindung zwischen den pneumatischen Bauteilen erfolgt per Schlauch. Die Verbindung der pneumatischen Bauteile mit der Elektronik (z.B. für Ventile und Drucksensoren) erfolgt per Kabel. Der Fertigungsaufwand ist hoch. Die Schläuche müssen zugeschnitten, aufgesteckt und gesichert werden. Die Kabel müssen konfektioniert werden. Diese Tätigkeiten können nicht maschinell durchgeführt werden. Durch die manuelle Fertigung und den monotonen Ablauf ist die Fehlerhäufigkeit hoch.

Gemäß dem zweiten Prinzip werden die pneumatischen Bauteile auf einem Block integriert. In diesem Block sind alle pneumatischen Verbindungen realisiert. Die pneumatischen Bauteile werden auf diesen Block aufgeschraubt. Eine Verschlauchung ist nicht oder kaum erforderlich. Die Verbindung der pneumatischen Bauteile mit der Elektronik wird über Kabel realisiert. Durch das zweite Prinzip wird die Verschlauchung mit all ihren Fehlermöglichkeiten eingespart.

Es ist bereits ein laparoskopischer Insufflator bekannt, der einen Zuleitungsanschluss, einen Ableitungsanschluss und eine zwischen diesen Anschlüssen angeordnete Druck- und Strömungsmesseinrichtung aufweist. Die Messeinrichtung dient zum Bestimmen des Gasdrucks und des Gasvolumenstroms am Ableitungsanschluss. Die Bauelemente sind als diskrete Teile innerhalb eines Gehäuses angeordnet (US 6,299,592 B1).

Weiterhin ist ein Gerät mit fluidischen und elektrischen Eigenschaften bekannt, das einen Strömungskanal in einem plattenartigen Modul aus Halbleitermaterial oder Glasmaterial aufweist (WO 2007/114.687 A1).

Ebenfalls bekannt sind fluidische Mikrosystem aus Leiterplatten, bei denen zwischen den Leiterplatten Hohlräume gebildet sind. Die Leiterplatten sind auf ihren einander zugewandten in die Hohlräume gerichteten Seiten mit Leiterbahnen versehen (DE 197 39722 A1).

Darüber hinaus ist es bekannt, auf einer aus mehreren einzelnen Platten bestehenden Platte Bauelemente anzubringen, die durch innerhalb der Platte verlaufende Kanäle miteinander verbunden sind. Die Platte besteht vorzugsweise aus Aluminium (EP 1 596 455 A1).

Ausgehend von bekannten Insufflatoren ist das Ziel der Erfindung, einen verbesserten Insufflator zu schaffen, der infolge einfachen Aufbaus wirtschaftlich gefertigt werden kann, zuverlässig ist und auch ausreichend hohe Volumenströme zulässt.

Erfindungsgemäß wird diese Aufgabe durch einen Insufflator gelöst, der in üblicher Manier einen Zuleitungsanschluss und einen Ableitungsanschluss und eine zwischen diesen Anschlüssen angeordnete Druck- und Strömungsmesseinrichtung aufweist. Die Druck- und Strömungsmesseinrichtung ist zum Bestimmen von einem am Ableitungsanschluss anliegenden Gasdruck und eines am Ableitungsanschluss anliegenden Gasvolumenstroms ausgebildet. Erfindungsgemäß umfasst die Druck- und Strömungsmesseinrichtung eine mehrschichtige Platine, die in ihrem Inneren wenigstens einen Strömungskanal aufweist, der an seinem Eingang mit dem Zuleitungsanschluss und an seinem Ausgang mit dem Ableitungsanschluss verbunden ist. Auf der Platine sind Druckmesssensoren sowie elektronische Bauelemente zur Beschaltung der Druckmesssensoren angeordnet. Die Druckmesssensoren sind jeweils durch eine entsprechende Öffnung mit einer den Strömungskanal im Übrigen dicht abdeckenden Platinenschicht mit dem Strömungskanal in unmittelbarer Verbindung und ausgebildet, einen am Ort der jeweiligen Öffnung herrschenden statischen Druck repräsentierenden Ausgangswert zu liefern.

Ein Vorteil der Erfindung besteht darin, dass die Platine, die den Strömungskanal einschließt, im Übrigen solchen Leiterplatten entspricht, wie sie in bekannten Geräten für elektronische Komponenten verwendet werden, so dass die elektronischen Komponenten über die Platine mit den ansonsten separat realisierten fluidischen Elementen eines Insufflators zu einer Baugruppe zusammengefasst werden. Dies führt in vorteilhafter Weise zu einer monolithischen Integration von Elektronik und Fluidik auf einer gemeinsamen Basis. Dadurch entfällt der sonst übliche Verschlauchungs- und Verdrahtungsaufwand. Dies wiederum vereinfacht die Herstellung und erhöht die Zuverlässigkeit. Die Herstellung kann auch einfach automatisiert werden. Schließlich kann der Insufflator - auch im Vergleich zu bekannten Insufflatoren - äußerst kompakt aufgebaut werden. Durch die Verwendung von Leiterplattenmaterial als Basis für die Platine des erfindungsgemäßen Insufflators können weiter Kosten reduziert werden. Leiterplatten und die dazugehörige Technologie sind weit verbreitet und sind sehr kostengünstig.

In einer bevorzugten Ausführungsform des Insufflators sind bei der mehrschichtigen Platine demnach alle Platinen herkömmliche glasfaserverstärkte Epoxidharzleiterplatten, beispielsweise solche mit einer Materialkennung FR4.

Allerdings ist die heute bekannte Standard-Leiterplattentechnologie nicht in der Lage, fluidische Funktionen zu übernehmen. Diese bisher geltende Beschränkung wird durch eine Platine überwunden, die in ihrem Inneren einen Strömungskanal aufweist und beispielsweise als mehrschichtige Platine aufgebaut sein kann.

Dies erlaubt - wie bereits gesagt - einen monolithischen Aufbau durch automatisierte Fertigung. Der gesamte CO₂-Insufflator, der auch das Netzteil einschließen kann, wird auf Basis einer mehrschichtigen Platine durch Automaten gefertigt. Es ist dann nur noch der Einbau in ein klinisch taugliches Gerät, die Kalibrierung und die Endkontrolle erforderlich.

Die so erzielte Vereinfachung kann zu einer Einsparung in der Größenordnung bis 70 % der Fertigungskosten heutiger Insufflatoren führen. Vorzugsweise sind der Zuleitungsanschluss und der Ableitungsanschluss sowie die Drucksensoren jeweils derart gasdicht auf die mehrschichtige Platine aufgeklebt, dass der Zuleitungsanschluss, der Ableitungsanschluss und die Drucksensoren mit dem von dem Hohlraum der Platine gebildeten Strömungskanal verbunden und somit pneumatisch miteinander verbunden sind. Durch das Aufkleben der fluidischen Komponenten (Zuleitungsanschluss, Ableitungsanschluss und Drucksensoren) auf die Platine ist es möglich, diese Komponenten gasdicht und fest mit der Platine zu verbinden. Ein geeigneter Klebstoff ist beispielsweise der Klebstoff, der auch zum Fixieren der üblichen elektronischen Komponenten auf der Platine vor dem Verlöten dieser Komponenten verwendet wird. Auf diese Weise ist eine besonders einfache automatische Fertigung möglich, die gleichzeitig zu einem qualitativ hochwertigen Ergebnis führt.

Alternativ sind der Zuleitungsanschluss und der Ableitungsanschluss sowie die Drucksensoren jeweils über Schrauben oder andere Befestigungsmittel mit der mehrschichtigen Platine verbunden.

Die Drucksensoren sind auf der mehrschichtigen Platine vorzugsweise so angeordnet, dass sie rückwärtig auf der Platine aufgeklebt sind und auf diese Weise mit dem Strömungskanal fluidverbunden sind. Als eine Konsequenz sind die Drucksensoren im Betrieb des Insufflators rückwärtig mit Druck beaufschlagt. Der Begriff "rückwärtig" ist hier so zu verstehen, dass diejenige Öffnung eines üblichen Differenz-Drucksensors, die üblicherweise mit der Umgebungsluft in unmittelbarer Verbindung steht, bei der bevorzugten Ausführungsvariante mit dem Strömungskanal in Verbindung steht, während die andere, üblicherweise dem zu messenden Druck zugewandte Öffnung eines üblichen Drucksensors zum Umgebungsdruck hin offen ist. Alternativ können die Drucksensoren auch umgekehrt angeordnet sein.

Vorzugsweise sind die Drucksensoren darüber hinaus mit ihren elektrischen Anschlüssen auf die Platine gelötet. Hierzu weist die Platine vorzugsweise elektrische Leiterbahnen auf. Damit ist eine weitere sichere mechanische und elektrische Verbindung dieser Drucksensoren mit der Platine und den übrigen elektronischen Komponenten des Insufflators gegeben. Diese übrigen Komponenten sind vorzugsweise ebenfalls auf die Platine gelötet und über Leiterbahnen auf der mehrschichtigen Platine miteinander und mit den Drucksensoren verbunden.

Außerdem sind vorzugsweise ein oder mehrere Ventile auf der Platine derart angebracht, dass sie mit den Strömungskanälen fluidverbunden und im Betrieb des Insufflators mit Druck und Flow beaufschlagt werden. Die Ventile weisen elektrische Anschlüsse auf, die vorzugsweise mit Leiterbahnen der Platine verlötet sind.

In einer bevorzugten Ausführungsform des Insufflators ist der Strömungskanal von einem Hohlraum in der mehrschichtigen Platine gebildet, der derart geformt ist, dass er einen Abschnitt aufweist, der als Strömungsdrossel wirkt und eine Volumenstrommessung nach dem Prinzip des Pneumotachographen erlaubt.

Für eine Volumenstrommessung weist die Strömungsdrossel in einer bevorzugten Ausführungsform beispielsweise folgende Dimensionen auf: Kanallänge von etwa 60 mm, Kanalhöhe von etwa 1 mm und einen Kanalbreite von etwa 6 mm.

Bevorzugt ist am Eingang und am Ausgang des als Strömungsdrossel wirkenden Abschnitts des Hohlraums jeweils ein Drucksensor angeordnet, der jeweils mit elektronischen Komponenten verbunden ist, wobei die Drucksensoren ausgebildet sind, eine Differenz zwischen dem statischen Druck am Eingang des als Strömungsdrossel wirkenden Abschnitts des Hohlraums und dem statischen Druck am Ausgang des als Strömungsdrossel wirkenden Abschnitts des Hohlraums zu bestimmen.

Der Insufflator ist demnach bevorzugt dazu ausgebildet, eine Strömungsregelung durch eine Druckregelung zu implementieren. Dazu ist der Insufflator in einer Ausführungsform ausgebildet, nach einem Niederdruckprinzip zu verfahren, bei dem der Insufflationsdruck gleich einem Solldruck ist, der in der Regel einem theoretischen maximalen Druck im Abdomen entspricht. Aufgrund eines Druckabfalls über einen Einlass und entlang einer Wandung eines zum Abdomen führenden Schlauches herrscht im Abdomen tatsächlich ein niedriger Druck als der Solldruck. Dieses Verfahren stellt meistens ein kontinuierliches Verfahren dar.

In einer anderen Ausführungsform ist der Insufflator ausgebildet, nach einem Überdruckprinzip zu arbeiten, bei dem der Insufflationsdruck stufenweise höher als der Solldruck eingestellt wird, wobei hier eine Messung eines intraabdominellen Drucks zyklisch in Pausen erfolgt, in denen der Insufflationsdruck und der Volumenstrom auf einen Betrag von jeweils etwa Null eingestellt werden. Dieses Überdruckverfahren stellt zumeist ein intermittierendes Verfahren dar.

In einer bevorzugten Ausführungsform ist der Insufflator ausgebildet, das Gas gemäß dem aus der Veröffentlichung der europäischen Patentanmeldung EP 1 352 669 A1 bekannten quasi-kontinuierlichen Verfahren in den Körper des Patienten einzuleiten. Auf diese Veröffentlichung, insbesondere auf die Ausführungsbeispiele gemäß den Figuren 4 und 5, wird hiermit explizit verwiesen. Demnach ist der Insufflator bevorzugt ausgebildet, die Insufflation des Gases in Abhängigkeit von einem vorgebbaren Zieldruck im Körper und dem Volumenstrom durchzuführen, bei einem Istdruck im Körper des Patienten kleiner als der Zieldruck das Gas mit Überdruck größer als der Zieldruck zu insufflieren, zum Ermitteln einer Differenz und einer Regelung zwischen Zieldruck und Istdruck im Körper den Volumenstrom während der Insufflation mit Überdruck zyklisch bis zum Zieldruck oder einem niedrigeren Solldruck zu reduzieren und den Wert des gemessenen Volumenstroms beim Zieldruck oder Solldruck als Regelgröße zwischen Zieldruck und Istdruck im Körper für die weitere Insufflation zu verwenden. Der Strömungskanal im Inneren der mehrschichtigen Platine wird vorzugsweise von einem Hohlraum gebildet, der eine lichte Höhe (in Dickenrichtung der Platine) von 1 mm und eine lichte Breite von 1 - 8 mm hat. Es hat sich herausgestellt, dass ein derartiger Strömungskanal zum einen leicht mit Hilfe einer mehrschichtigen Platine zu realisieren ist und im Übrigen einen Volumenstrom von mehr als 40 l/min gasförmigem CO₂ zulässt. Entsprechend wird auch ein Insufflator bevorzugt, der für einen CO₂-Gasvolumenstrom von mehr als 40 l/min ausgebildet ist.

Außerdem ist der Insufflator vorzugsweise für einen Ausgangsdruck (der dem Abdominaldruck entspricht) von bis zu 30 mm_{Hg} ausgebildet. Dies entspricht in etwa einem Druck von 4 kPa.

Ein Beatmungsgerät, ein Überwachungs- oder Patientenmonitoring-Gerät und ein Blutdruckmessgerät bilden weitere Aspekte der Erfindung. Das Beatmungsgerät, Überwachungs- oder Patientenmonitoring-Gerät und Blutdruckmessgerät weisen jeweils in üblicher Manier einen Zuleitungsanschluss und einen Ableitungsanschluss und eine zwischen diesen Anschlüssen angeordnete Druck- und Strömungsmesseinrichtung auf. Die Druck- und Strömungsmesseinrichtung ist zum Bestimmen von einem am Ableitungsanschluss anliegenden Gasdruck und eines am Ableitungsanschluss anliegenden Gasvolumenstroms ausgebildet. Erfindungsgemäß umfasst die Druck- und Strömungsmesseinrichtung eine mehrschichtige Platine, die in ihrem Inneren wenigstens einen Strömungskanal aufweist, der an seinem Eingang mit dem Zuleitungsanschluss und an seinem Ausgang mit dem Ableitungsanschluss verbunden ist. Auf der Platine sind Druckmesssensoren sowie elektronische Bauelemente zur Beschaltung der Druckmesssensoren angeordnet. Die Druckmesssensoren sind jeweils durch eine entsprechende Öffnung mit einer den Strömungskanal im Übrigen dicht abdeckenden Platinenschicht mit dem Strömungskanal in unmittelbarer Verbindung und ausgebildet, einen am Ort der jeweiligen Öffnung herrschenden statischen Druck repräsentierenden Ausgangswert zu liefern.

Das Beatmungsgerät, das Überwachungs- bzw. Patientenmonitoring-Gerät und das Blutdruckmessgerät teilen weitestgehend die Vorteile des erfindungsgemäßen Insufflators.

Gegenstand der vorliegenden Erfindung ist auch ein Verfahren zum Herstellen eines Insufflators, welches die Schritte umfasst:
- Bereitstellen einer mehrschichtigen Platine mit integriertem Strömungskanal;
- Bestücken der Platine mit fluidischen Bauelementen durch Aufkleben der fluidischen Bauelemente;
- Bestücken der Platine mit elektrischen Bauelementen durch Auflöten der elektrischen Bauelemente.

Vorzugsweise umfasst der Verfahrensschritt des Bestückens der Platine mit fluidischen Bauelementen das Auftragen von Klebstoff mittels eines Dispensers. Vorzugsweise erfolgt dieser Kleberauftrag in der bei einer Bestückung von elektronischen Bauelementen in SMD-Technik (SMD: Surface Mounted Device) üblichen Manier.

Weitere vorteilhafte Ausgestaltungen des erfindungsgemäßen Insufflators ergeben sich aus der nachfolgenden Beschreibung eines Ausführungsbeispiels.

Die Erfindung soll nun anhand eines Ausführungsbeispiels mit Bezug auf die Figuren näher erläutert werden. Von den Figuren zeigt:
- Fig. 1:: Einen schematischen Querschnitt durch einen erfindungsgemäßen Insufflator;
- Fig. 2:: eine schematische Darstellung einer Aufsicht auf eine bestimmte mehrschichtige Platine des erfindungsgemäßen Insufflators; und
- Fig. 3:: in schematischer Darstellung das prinzipielle Layout der Platine aus Fig. 2.

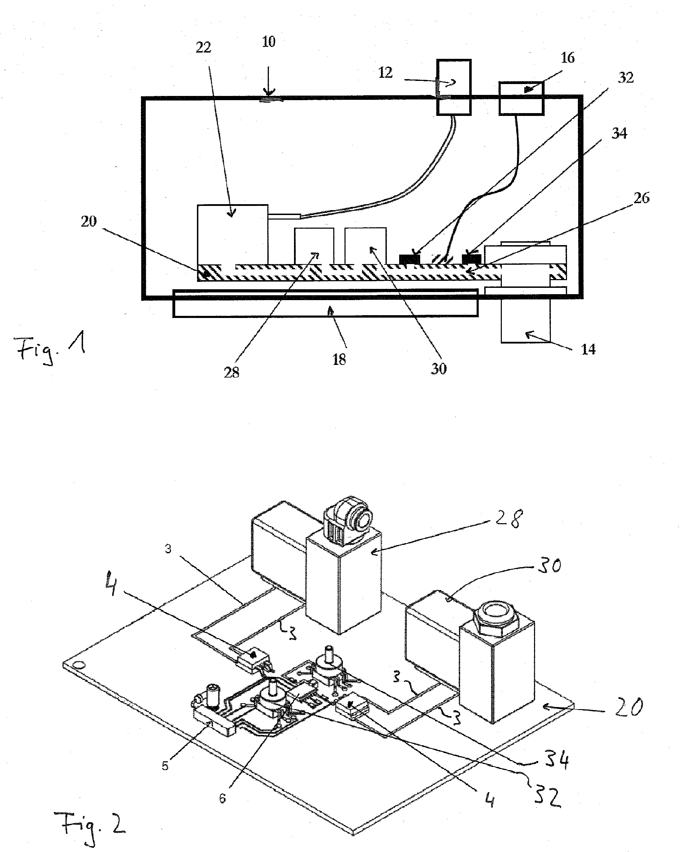

In Fig. 1 ist schematisch ein Gehäuse 10 eines Insufflators angedeutet, an welchem ein CO₂-Anschluss 12 zum Anschluss an eine Quelle für gasförmiges CO₂ vorgesehen ist sowie einen Ableitungsanschluss 14, an den ein Schlauch zum Einführen in das Abdomen eines Patienten anzuschließen ist. Außerdem weist der Insufflator gemäß Fig. 1 einen elektrischen Netzanschluss 16 sowie ein Bedienpanel 18 auf.

Kernstück des Insufflators aus Fig. 1 ist eine mehrschichtige Platine 20, auf die ein Zuleitungsanschluss 22 und der Ableitungsanschluss 14 gasdicht aufgeklebt sind. Der Zuleitungsanschluss 22 ist mit dem CO₂-Anschluss 12 über einen entsprechenden Schlauch 11 verbunden.

Die Platine 20 ist mehrschichtig und weist in ihrem Inneren Hohlräume auf, die einen mehrteiligen Strömungskanal 26 bilden. Der Zuleitungsanschluss 22 und der Ableitungsanschluss 14 sind mit diesem Strömungskanal 26 fluidverbunden. Außerdem sind auf der Außenseite der Platine 20 elektrische und elektronische Bauteile des Insufflators montiert sowie Ventile 28 und 30 und Drucksensoren 32 und 34. Die Ventile 28 und 30 und die Drucksensoren 32 und 34 sind ebenfalls gasdicht auf die Platine 20 aufgeklebt. Die Drucksensoren 32 und 34 sind Differenzdrucksensoren, die rückwärtig auf die Platine 20 gasdicht aufgeklebt sind.

Die verschiedenen Abschnitte des Strömungskanals 26 im Inneren der Platine 20 haben eine lichte Höhe von 1 mm und eine lichte Breite zwischen 1 mm und 8 mm.

Der Insufflator erlaubt einen Gasvolumenstrom von CO₂ von bis zu 44 l/min. Er ist für einen Intraabdominaldruck von 1 bis 30 mm_{Hg} ausgelegt. Durch die Ventile 28 und 30 ist ein intermittierender Gasfluss möglich.

Fig. 2 zeigt in schematischer Darstellung das Kernstück des Insufflators aus Fig. 1, nämlich die mehrschichtige Platine 20 mit den darauf montierten elektronischen und fluidischen Bauelementen, die für den beispielhaft dargestellten Insufflator erforderlich sind. Dies sind die Ventile 28 und 30, leistungselektronische Bauelemente 4, welche mit den Ventilen 28 bzw. 30 über Leiterbahnen 3 verbunden sind, die Drucksensoren 32 und 34, einen Steckverbinder 5 zum Verbinden mit dem in Fig. 1 nicht dargestellten Netzanschluss 16, , eine Ventilansteuerung, eine Sensorsignalaufbereitung, ein Mikrocontroller 6, Tasten und Anzeigen. Die Strömungskanäle im Inneren der mehrschichtigen Platine sind in Fig. 2 nicht zu erkennen. Sie stellen die fluidische Verbindung zwischen dem Zuleitungsanschluss 22 und dem Ableitungsanschluss 14 sowie den Ventilen 28 und 30 und Drucksensoren 32 und 34 dar, die als pneumatische (fluidische) Bauelemente ebenfalls auf der Platine 20 befestigt sind. Die fluidischen Bauelemente sind dabei so angeordnet, dass sie sowohl fluidischen Kontakt zu den im Inneren der Platine 20 angeordneten Strömungskanälen als auch elektrischen Kontakt zu den elektrischen und elektronischen Bauelementen 4 und 6 des Insufflators haben. Diese doppelte Nutzung der Platine 20 ermöglicht einen kompakten und preiswerten Aufbau eines Insufflators.

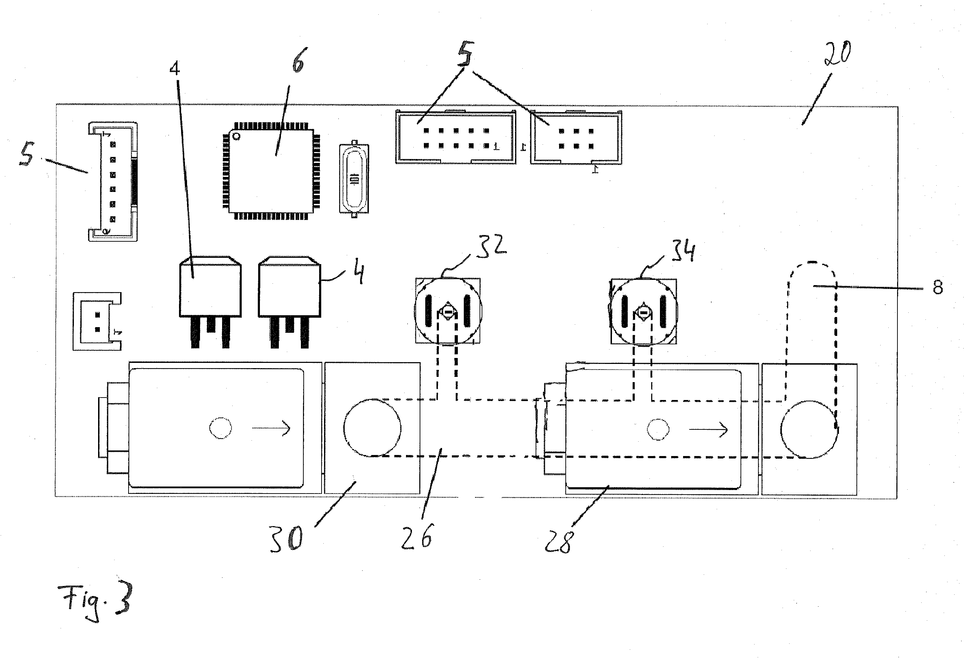

In Fig. 3 zeigt in schematischer Darstellung das Layout der Platine 20 einschließlich des Strömungskanals 26, der leistungselektronischen Bauelemente 4, des Mikrocontrollers 6, einer Vielzahl Steckverbinder 5, der montierten Ventile 28 und 30, der Drucksensoren 32 und 34 und einem Strömungskanalausgangs 8, welcher zum in Fig. 3 nicht dargestellten Ableitungsanschluss 14 führt. Diese mehrschichtige Platine 20 stellt eine wichtige Neuerung des erfindungsgemäßen Insufflators dar. Entscheidend hierfür sind die Hohlräume in Inneren der Platine 20, die dadurch erzeugt werden, dass einzelne Leiterplatten mit geeignetem Layout großflächig und gasdicht miteinander verklebt werden. Eine derartige mehrschichtige Platine weicht von bekannten Standardtechnologien für die Herstellung von Multilayer-Leiterplatten ab, da die einzelnen Leiterplatten zunächst vollständig mit einem geeigneten Klebstoff beschichtet und anschließend verpresst werden müssen.

Bekannte Verklebungstechniken von bekannten Multilayer-Leiterplatten unterscheiden sich von der erfindungsgemäßen mehrschichtigen Platine dadurch, dass sie üblicherweise nicht gasdicht miteinander verklebt sind, während dies bei der mehrschichtigen Platine 20 erfindungsgemäß der Fall ist.

### Bezugszeichenliste

- 3: Leiterbahnen
- 4: leistungselektronische Bauelemente
- 5: Steckverbinder
- 6: Mikrocontroller
- 8: Ausgang des Strömungskanals 26
- 10: Gehäuse eines Insufflators
- 11: Schlauch
- 12: CO₂-Anschluss
- 14: Ableitungsanschluss
- 16: Netzanschluss
- 18: Bedienpanel
- 20: mehrschichtige Platine
- 22: Zuleitungsanschluss
- 26: Strömungskanal
- 28,30: Ventile
- 32,34: Drucksensoren

## Patentansprüche

1. Insufflator mit einem Zuleitungsanschluss (22) und einem Ableitungsanschluss (14) und einer zwischen diesen Anschlüssen (22, 14) angeordneten Druck- und Strömungsmesseinrichtung zum Bestimmen von einen am Ableitungsanschluss (14) anliegenden Gasdruck und einen am Ableitungsanschluss (14) anliegenden Gasvolumenstrom charakterisierenden Messgrößen,
**dadurch gekennzeichnet, dass** die Druck- und Strömungsmesseinrichtung eine mehrschichtige Platine (20) umfasst, die in ihrem Inneren wenigstens einen Strömungskanal (26) umfasst, der an seinem Eingang mit dem Zuleitungsanschluss (22) und an seinem Ausgang (8) mit dem Ableitungsanschluss (14) verbunden ist,
wobei auf der Platine (20) Druckmesssensoren (32, 34) sowie elektronische Bauelemente (4, 6) zur Beschaltung der Druckmesssensoren (32, 34) angeordnet sind, von denen die Druckmesssensoren (32, 34) jeweils durch eine entsprechende Öffnung einer den Strömungskanal (26) im Übrigen dicht abdeckenden Platinenschicht mit dem Strömungskanal (26) in unmittelbarer Verbindung stehen und ausgebildet sind, einen den am Ort der jeweiligen Öffnung herrschenden statischen Druck repräsentierenden Ausgangswert zu liefern.

2. Insufflator nach Anspruch 1, **dadurch gekennzeichnet, dass** der Zuleitungsanschluss (22) und der Ableitungsanschluss (14) sowie die Drucksensoren (32, 34) jeweils derart gasdicht auf die den Strömungskanal (26) bedeckende Schicht der Platine (20) aufgeklebt sind, dass der Zuleitungsanschluss (22), der Ableitungsanschluss (14) und die Drucksensoren (32, 34) über den Strömungskanal (26) pneumatisch miteinander verbunden sind.

3. Insufflator nach Anspruch 2, **dadurch gekennzeichnet, dass** die Drucksensoren (32, 34) rückwärtig mit dem Strömungskanal (26) fluidverbunden und im Betrieb des Insufflators rückwärtig mit Druck beaufschlagt sind.

4. Insufflator nach einem der Ansprüche 1 bis 3, **dadurch gekennzeichnet, dass** die Drucksensoren (32, 34) mit ihren elektrischen Anschlüssen auf die Platine (20) gelötet sind.

5. Insufflator nach einem der Ansprüche 1 bis 4, **gekennzeichnet durch** wenigstens ein Ventil (28, 30), das mit dem Strömungskanal (26) derart fluidverbunden ist, dass es im Betrieb des Insufflators mit Druck und einem Gasvolumenstrom beaufschlagt wird.

6. Insufflator nach Anspruch 5, **dadurch gekennzeichnet, dass** das Ventile (28, 30) elektrische Anschlüsse aufweist, die auf die Platine (20) gelötet sind.

7. Insufflator nach einem der Ansprüche 1 bis 5, **dadurch gekennzeichnet, dass** der Strömungskanal (26) von einem Hohlraum in der mehrschichtigen Platine (20) gebildet ist, der derart geformt ist, dass er einen Abschnitt aufweist, der als Strömungsdrossel wirkt und eine Volumenstrommessung nach dem Prinzip des Pneumotachographen erlaubt.

8. Insufflator nach Anspruch 7, **dadurch gekennzeichnet, dass** am Eingang und am Ausgang (8) des als Strömungsdrossel wirkenden Abschnitts des Hohlraums jeweils ein Drucksensor (32, 34) angeordnet ist, die mit elektronischen Komponenten verbunden (4, 6) sind, die ausgebildet sind, eine Differenz zwischen dem statischen Druck am Eingang des als Strömungsdrossel wirkenden Abschnitts des Hohlraums und dem statischen Druck am Ausgang des als Strömungsdrossel wirkenden Abschnitts des Hohlraums zu bestimmen.

9. Insufflator nach einem der Ansprüche 1 bis 8, **dadurch gekennzeichnet, dass** der Strömungskanal (26) eine lichte Höhe von 1 mm und eine lichte Breite von 1 mm bis 8 mm hat.

10. Insufflator nach einem der Ansprüche 1 bis 9, **dadurch gekennzeichnet, dass** der Insufflator für einen CO₂ Gasvolumenstrom von mehr als 40 l/min ausgebildet ist.

11. Insufflator nach einem der Ansprüche 1 bis 10, **dadurch gekennzeichnet, dass** der Insufflator für einen Eingangsgasdruck (Abdominaldruck) von bis zu mindestens 30 mm_{Hg} ausgebildet ist.

12. Verfahren zum Herstellen eines Insufflators nach einem der vorhergehenden Ansprüche, welches die Schritte umfasst:
- Bereitstellen einer mehrschichtigen Platine (20) mit integriertem Strömungskanal (26);
- Bestücken der Platine (20) mit fluidischen Bauelementen durch Aufkleben der fluidischen Bauelemente;
- Bestücken der Platine (20) mit elektronischen Bauelementen (4, 6) durch Auflöten der elektronischen Bauelemente (4, 6).

13. Verfahren nach Anspruch 12, bei dem der Verfahrensschritt des Bestückens der Platine (20) mit fluidischen Bauelementen das Auftragen von Klebstoff mittels eines Dispensers einschließt.

14. Verfahren nach Anspruch 13, bei dem der Auftrag von Klebstoff in der bei einer Bestückung von elektronischen Bauelementen (4, 6) in SMD-Technik üblichen Manier erfolgt.

## Claims

1. Insufflator with a supply line connection (22) and a discharge line connection (14) and a pressure and flow measuring device arranged between these connections (22, 14) for determining a gas pressure applied to the discharge line connection (14) and a gas volume flow applied to the discharge line connection (14) characterizing measurement variables,
**characterized in that** the pressure and flow measuring device comprises a multilayer circuit board (20) which comprises in its interior at least one flow channel (26) which is connected at its inlet to the supply line connection (22) and at its outlet to the discharge line connection (14),
wherein pressure measuring sensors (32, 34) and electronic components (4, 6) for wiring the pressure measuring sensors (32, 34) are arranged on the board (20), of which the pressure measuring sensors (32, 34) are each in direct communication with a flow channel (26) via a corresponding opening of the flow channel (26) in the otherwise tightly covering circuit board layer and are formed to provide an initial value representing the static pressure prevailing at the location of the respective opening.

2. Insufflator according to claim 1, **characterized in that** the supply line connection (22) and the discharge line connection (14) as well as the pressure sensors (32, 34) are each glued in a gas-tight manner onto the layer of the circuit board (20) covering the flow channel (26) in such a way that the supply line connection (22), the discharge line connection (14) and the pressure sensors (32, 34) are pneumatically connected to one another via the flow channel (26).

3. Insufflator according to claim 2, **characterized in that** the pressure sensors (32, 34) are fluidly connected to the flow channel (26) at the rear and are subjected to pressure at the rear during operation of the insufflator.

4. Insufflator according to any of claims 1 to 3, **characterized in that** the pressure sensors (32, 34) are soldered with their electrical connections to the circuit board (20).

5. Insufflator according to any of claims 1 to 4, **characterized by** at least one valve (28, 30) which is fluidly connected to the flow channel (26) in such a way that it is subjected to pressure and a gas volume flow during operation of the insufflator.

6. Insufflator according to claim 5, **characterized in that** the valve (28, 30) has electrical terminals which are soldered to the circuit board (20).

7. Insufflator according to any of claims 1 to 5, **characterized in that** the flow channel (26) is formed by a cavity in the multilayer circuit board (20) which is shaped in such a way that it has a section which acts as a flow throttle and permits volume flow measurement according to the principle of the pneumatic tachograph.

8. Insufflator according to claim 7, **characterized in that** in each case one pressure sensor (32, 34) is arranged at the inlet and at the outlet (8) of the section of the cavity acting as a flow restrictor and is connected to electronic components (4, 6) which are designed to determine a difference between the static pressure at the inlet of the section of the cavity acting as a flow restrictor and the static pressure at the outlet of the section of the cavity acting as a flow restrictor.

9. Insufflator according to any of claims 1 to 8, **characterized in that** the flow channel (26) has a clear height of 1 mm and a clear width of 1 mm to 8 mm.

10. Insufflator according to any of claims 1 to 9, **characterized in that** the insufflator is designed for a CO₂ gas volume flow of more than 40 l/min.

11. Insufflator according to any of claims 1 to 10, **characterized in that** the insufflator is designed for an inlet gas pressure (abdominal pressure) of up to at least 30 mmHg.

12. Method of manufacturing an insufflator according to any of the previous claims, which comprises the steps of:
- Providing a multilayer circuit board (20) with an integrated flow channel (26);
- Equipping the circuit board (20) with fluidic components by bonding the fluidic components;
- Equipping the circuit board (20) with electronic components (4, 6) by soldering on the electronic components (4, 6).

13. Method according to claim 12, wherein the step of equipping the board (20) with fluidic components includes applying adhesive by means of a dispenser.

14. Method according to claim 13, wherein the application of adhesive is carried out in the usual manner for the assembly of electronic components (4, 6) in SMD technology.

## Revendications

1. Insufflateur, pourvu d'un raccord d'alimentation (22) et d'un raccord d'évacuation (14) et d'un système manomètre et débitmètre placé entre lesdits raccords (22, 14), destiné à déterminer une pression gazeuse appliquée sur le raccord d'évacuation (14) et un débit volumétrique gazeux appliqué sur le raccord d'évacuation (14), **caractérisé en ce que** le système manomètre et débitmètre comprend un circuit imprimé (20) multicouches, qui dans son intérieur comprend au moins un canal d'écoulement (26) qui sur son entrée est relié avec le raccord d'alimentation (22) et sur sa sortie (8) est relié avec le raccord d'évacuation (14), sur le circuit imprimé (20) étant placés des capteurs de mesure de la pression (32, 34), ainsi que des composants électroniques (4, 6), destinés à mettre en circuit les capteurs de mesure de la pression (32, 34) par lesquels les capteurs de mesure de la pression (32, 34) sont en liaison directe avec le canal d'écoulement (26), chaque fois à travers un orifice correspondant d'une couche de circuit imprimé recouvrant en outre de manière étanche le canal d'écoulement (26) et étant conçus pour fournir une valeur de départ représentant une pression statique régnant à l'endroit de l'orifice en question.

2. Insufflateur selon la revendication 1, **caractérisé en ce que** le raccord d'alimentation (22) et le raccord d'évacuation (14), ainsi que les capteurs de pression (32, 34) sont collés chacun de manière étanche au gaz sur la couche du circuit imprimé (20) recouvrant le canal d'écoulement (26), de telle sorte que le raccord d'alimentation (22), le raccord d'évacuation (14) et les capteurs de pression (32, 34) soient reliés par voie pneumatique les uns avec les autres par l'intermédiaire du canal d'écoulement (26).

3. Insufflateur selon la revendication 2, **caractérisé en ce que** sur l'arrière, les capteurs de pression (32, 34) sont reliés par fluide avec le canal d'écoulement (26) et en service de l'insufflateur, sont soumis par l'arrière à de la pression.

4. Insufflateur selon l'une quelconque des revendications 1 à 3, **caractérisé en ce que** par leurs raccordements électriques, les capteurs de pression (32, 34) sont brasés sur le circuit imprimé (20).

5. Insufflateur selon l'une quelconque des revendications 1 à 4, **caractérisé par** au moins une soupape (28, 30), qui est reliée par fluide avec le canal d'écoulement (26), de telle sorte qu'en service de l'insufflateur, elle soit soumise à de la pression et à un débit volumétrique gazeux.

6. Insufflateur selon la revendication 5, **caractérisé en ce que** la soupape (28, 30) comporte des raccordements électriques, qui sont brasés sur le circuit imprimé (20) .

7. Insufflateur selon l'une quelconque des revendications 1 à 5, **caractérisé en ce que** le canal d'écoulement (26) est formé par une cavité dans le circuit imprimé (20) multicouches qui est façonnée de telle sorte qu'elle comporte un tronçon qui fait effet d'organe d'étranglement et qu'elle permette une mesure du débit volumétrique d'après le principe du pneumotachographe.

8. Insufflateur selon la revendication 7, **caractérisé en ce que** sur l'entrée et sur la sortie (8) du tronçon de la cavité faisant office d'organe d'étranglement est placé chaque fois un capteur de pression (32, 34) qui sont reliés avec des composants électroniques (4, 6) qui sont conçus pour déterminer une différence entre la pression statique sur l'entrée du tronçon de la cavité faisant office d'organe d'étranglement et la pression statique sur la sortie du tronçon de la cavité faisant office d'organe d'étranglement.

9. Insufflateur selon l'une quelconque des revendications 1 à 8, **caractérisé en ce que** le canal d'écoulement (26) dispose d'une hauteur libre de 1 mm et d'une largeur libre de 1 mm à 8 mm.

10. Insufflateur selon l'une quelconque des revendications 1 à 9, **caractérisé en ce que** l'insufflateur est conçu pour un débit volumétrique gazeux de CO₂ de plus de 40 l/mn.

11. Insufflateur selon l'une quelconque des revendications 1 à 10, **caractérisé en ce que** l'insufflateur est conçu pour une pression gazeuse d'admission (pression abdominale) de jusqu'à au moins 30 mm_{Hg}.

12. Procédé, destiné à fabriquer un insufflateur selon l'une quelconque des revendications précédentes, lequel comprend les étapes consistant à :
- mettre à disposition un circuit imprimé (20) multicouches, pourvu d'un canal d'écoulement (26) ;
- équiper le circuit imprimé (20) de composants fluidiques, par collage des composants fluidiques ;
- équiper le circuit imprimé (20) de composants électroniques (4, 6) par brasage des composants électroniques (4, 6).

13. Procédé selon la revendication 12, lors duquel l'étape de procédé consistant à équiper le circuit imprimé (20) de composants fluidiques inclut l'application d'agent adhésif au moyen d'un distributeur.

14. Procédé selon la revendication 13, lors duquel l'application d'agent adhésif s'effectue de la manière habituelle lors de l'équipement de composants électroniques (4, 6) par technique SMD.
